Europäisches Patentamt

(19) European Patent Office

Office européen des brevets

(11) Publication number: **0 154 576**

A1

(12) # EUROPEAN PATENT APPLICATION

(21) Application number: **85400191.4**

(22) Date of filing: **05.02.85**

(51) Int. Cl.⁴: **C 12 N 15/00**
C 12 P 19/34, C 12 P 21/00
C 07 K 1/00, A 61 K 45/02

(30) Priority: **06.02.84 US 577291**

(43) Date of publication of application:
**11.09.85 Bulletin 85/37**

(84) Designated Contracting States:
**AT BE CH DE FR GB IT LI LU NL SE**

(71) Applicant: **INTERFERON SCIENCES, INC a Delaware
Corporation
375 Park Avenue Suite 2201
New York New York 10152(US)**

(72) Inventor: **Lee, Nancy
1113 Landing Lane
Princeton Junction New Jersey 08850(US)**

(72) Inventor: **Wainwright, Norman R.
10 Yorktown Road
Millstone New Jersey 08876(US)**

(74) Representative: **Sauvage, Renée
Cabinet Sauvage 100 bis, avenue de Saint-Mandé
F-75012 Paris(FR)**

(54) Hybrid nucleic acid sequences having tandemly arranged genes.

(57) A hybrid nucleic acid sequence for use in producing one or more selected polypeptides in a host cell is provided which comprises: a promoter sequence recognized by the host cell at one end; a transcription terminator sequence recognized by the host cell at the other end; and between the promoter sequence and the transcription terminator sequence: a first region which includes a ribosome-binding site, a translation initiation site, a coding region for one of said one or more selected polypeptides, and a translation termination site, but which does not include the promoter sequence or the transcription terminator sequence; and one or more second regions, each of which includes a ribosome-binding site, a translation initiation site, a coding region for the same or a different one of said one or more selected polypeptides, and a translation termination site, and which does not include the promoter sequence or the transcription terminator sequence, the reading directions of the promoter sequence, the transcription terminator sequence, the first region and each of the second regions having the same orientation.

## HYBRID NUCLEIC ACID SEQUENCES HAVING
## TANDEMLY ARRANGED GENES

### BACKGROUND OF THE INVENTION

#### 1. Field of the Invention

This invention relates to hybrid nucleic acid sequences for use in genetic engineering and in particular to hybrid sequences having tandemly arranged genes.

#### 2. Description of the Prior Art

One of the purposes of genetic engineering is to increase the production of desired gene products in a cell. Gene expression can be enhanced at the levels of transcription and translation by gene manipulation. Moreover, for genes located in plasmids, it has been found that the amount of a gene product produced by a cell can also be raised by either increasing the copy number of the plasmid harboring the gene, such as, by using a temperature-regulated runaway plasmid, or by increasing the copy number of the gene-promoter complex in the plasmid. See Uhlin, B., Molin, S., Gustafsson, P. and Nordstrom, K. (1979) Gene, 6:91-106, and Lee, N., Nakamura, K., Inouye, M. (1981) J. Bacterial., 146:861-866.

Theoretically, the higher the copy number of a cloned gene a plasmid contains, the higher will be the gene dosage effect. However, there are certain constraints. If the copy number is too high, as in the case of runaway plasmids, the cell may die as a result of overdosage or disruption of essential cell activities.

**0154576**

Moreover, as plasmid size gets larger, transformation efficiency of many cell types becomes lower, and larger sized plasmids tend to have low copy numbers. See Kushner, S.R. in Genetic Engineering, Boyer, H.W. and Nicosia, S. (Ed.), 1978, pp. 17-23, Elsevier/North Holland Biomedical Press, Amsterdam, and Broda, P. (1979) in Plasmids, W. H. Freeman and Co. Ltd., San Francisco. In particular, it has been found for many cell types that transformation efficiency decreases once the size of a plasmid increases above 15 Kb. See Rodriguez, R. and Tait, R. in Recombinant DNA Techniques, p. 4, Addison Wesley, Reading, Massachusetts (1983). Accordingly, for these cell types, plasmid length must be carefully controlled.

Up to now, hybrid plasmids and other genetically engineered hybrid nucleic acid sequences constructed for production of a particular gene product have contained at least the following regions or sites: a promoter region; a ribosome-binding region; a translation initiation region; a coding region for the gene of interest; a translational termination region; and a transcriptional termination region. (Note that the terms "region" and "site" are using interchangeably herein to describe nucleic acid sequences having particular functions, whether those sequences include a few residues, as in the case of a typical translation initiation or termination site, or many residues, as in the case of a typical promoter or ribosome-binding site.)

With a strong promoter and a fine tuned ribosome-binding region, this kind of construction will result in high levels of gene expression. However, if this scheme is used in cloning multiple copies of a gene so as to obtain the gene dosage effect, for every structural gene cloned, a promoter region and a transcriptional termination region also have to be cloned. The presence of multiple copies of these regions contributes significantly to the overall size of, for example, the hybrid plasmid used for cloning and thus leads to the transformation efficiency and low copy number problems described above for larger sized plasmids.

## SUMMARY OF THE INVENTION

In view of the above state of the art, it is an object of this invention to provide hybrid nucleic acid sequences which carry a plurality of selected genes and yet are smaller in size than prior art hybrid sequences carrying the same number of genes. In this way, the gene dosage effect can be enhanced since more copies of selected genes can be packed into host cells without increasing the number or size of the hybrid sequences introduced into the cells. Also, by close packing different but related genes, e.g., genes coding for the constituent proteins making up a biologically active molecule or for related proteins which in combination have a synergistic biological effect, improved final products and more efficient production can be obtained without sacrificing the gene dosage effect.

To achieve these and other objects, the invention, in accordance with one of its aspects, provides hybrid nucleic acid sequences in which multiple copies of a single gene are tandemly arranged, i.e., arranged one after the other, and placed under the control of a single promoter and a single transcriptional terminator. Each gene has associated with it a ribosome-binding site, a translacion initiation site and a translation termination site. Significantly, each gene does not have its own copy of the promoter and transcriptional terminator regions, thus producing the desired reduction in overall size of the hybrid nucleic acid sequence.

The genes are arranged in the hybrid sequence so that they all have the same reading direction.

In accordance with another of its aspects, the invention provides hybrid nucleic acid sequences in which a plurality of genes coding for different polypeptides are tandemly arranged and placed under the control of a single promoter and a single transcriptional terminator. Again, the genes are arranged in the same reading direction and each gene has its own ribosome-binding site, translation initiation site, and translation termination site, but does not have its own copy of the promoter and the transcriptional terminator. Hybrid nucleic acid sequences prepared in accordance with this embodiment when introduced into a host cell function in a manner similar to that of polycistronic bacterial operons. Such naturally occurring operons include several different genes which are translated as a single message. Examples include the lac operon which contains 3 linked genes (lac Z, lac Y, and lac A) and the trp operon which contains 5 linked genes (trp E, trp D, trp C, trp B and trp A). See Miller, J. H. and Reznikoff, W. S. (eds.) (1980) The Operon, 2nd ed. (Cold Spring Harbour Laboratory, New York).

In accordance with a further embodiment of the invention, a method for combining nucleic acid sequences so that their reading directions have the same orientation is provided comprising the steps of:

(a) preparing or selecting sequences which at one end have a ligatable end of a first type and at the other end have a ligatable end of a second type, the reading direction of a sequence being from the ligatable end of the first type towards the ligatable end of the second type, the union of two ligatable ends of the first type being susceptible to cleavage by a first restriction enzyme, the union of two ligatable ends of the second type being susceptible to cleavage by a second restriction enzyme, the union of a ligatable end of the first type with a ligatable end of the second type not being substantially susceptible to cleavage by either the first or the second restriction enzyme;

(b) ligating the sequences; and

(c) treating the ligated sequences with the first and second restriction enzymes.

In accordance with an additional embodiment of the invention, a method is provided for combining nucleic acid sequences of a first type with nucleic acid sequences of a second type to form hybrid sequences in which the reading directions of the the first and second sequences have the same orientation, and in which the number of first and second sequences are approximately equal, comprising the steps of:

(a) preparing or selecting sequences of the first type which at one end have a ligatable end of a first type and at the other end have a ligatable end of a second type, the reading direction of a sequence being from the ligatable end of the first type towards the ligatable end of the second type, the ligatable end of the first type not being substantially ligatable with the ligatable end of the second type, the union of two ligatable ends of the first type being susceptible to cleavage by a first restriction enzyme, the union of two ligatable ends of the second type being susceptible to cleavage by a second restriction enzyme;

(b) preparing or selecting sequences of the second type which at one end have a ligatable end of a third type and at the other end have a ligatable end of a fourth type, the reading direction of a sequence being from the ligatable end of the third type towards the ligatable end of the fourth type, the ligatable end of the third type not being substantially ligatable with the ligatable end of the fourth type, the ligatable end of the first type not being substantially ligatable with the ligatable end of the third type, the ligatable end of the second type not being substantially ligatable with the ligatable end of the fourth type, the union of two ligatable ends of the third type being susceptible to cleavage by a third restriction enzyme, the union of two ligatable ends of the fourth type being susceptible to cleavage by a fourth restriction enzyme, the union of a ligatable end of the first type with a ligatable end of the fourth type and the union of a ligatable end of the second type with a ligatable end of the third type not being substantially susceptible to cleavage by any of the first, second, third or fourth restriction enzymes;

(c) ligating the sequences of the first and second types with each other; and

(d) treating the ligated sequences with the first, second, third and fourth restriction enzymes.

In certain preferred embodiments of the invention, the tandem gene technology of the present invention is used to produce polypeptides which display immunological and biological activities of human leukocyte interferon.

BRIEF DESCRIPTION OF THE DRAWING

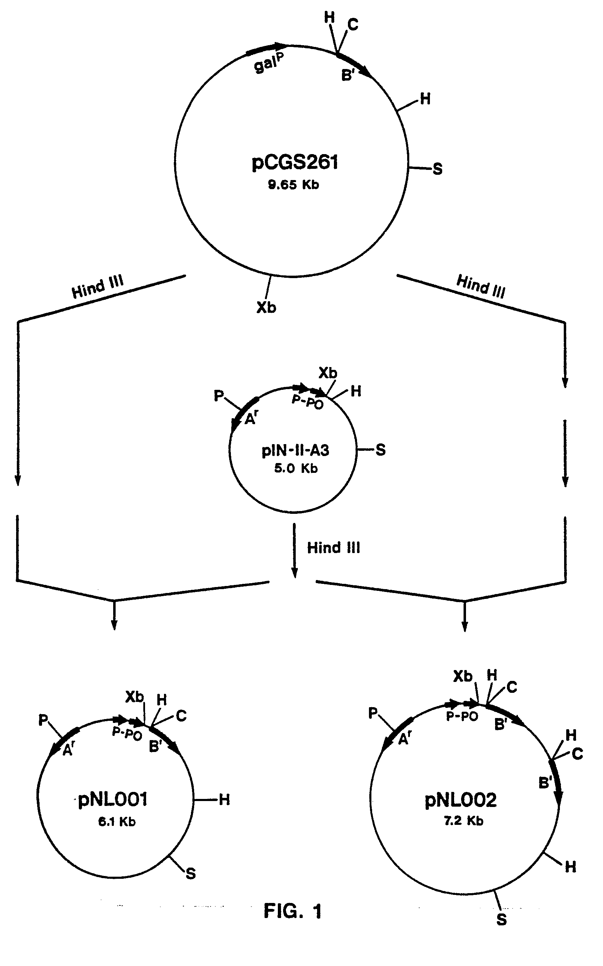

Fig. 1 schematically shows a process for cloning either one or two copies of an INF-B' containing Hind III fragment (1.1Kb) into the inducible expression vehicle pIN-II-A3. pNL001 contains one Hind III fragment, while pNL002 contains two Hind III fragments in tandem. Abbreviations used: $gal^P$ = yeast galactose promoter, P-PO = lipoprotein promoter + lac promoter-operator, $A^r$ = ampicillin resistance, B' = interferon B' gene, Xb = XbaI, H = Hind III, C = ClaI, S = SalI, P = PstI.

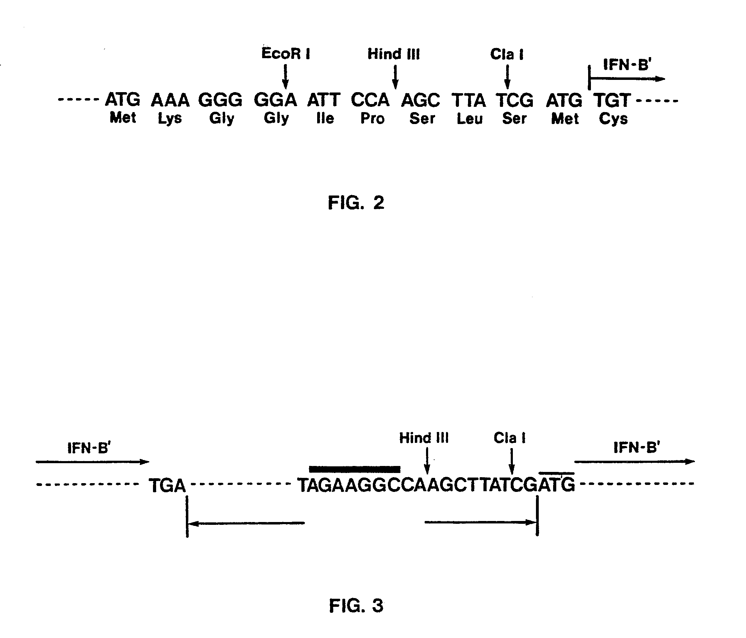

Fig. 2 shows the N-terminus of the coding region immediately 3' to the lipoprotein promoter or lactose promoter-operator of the hybrid interferon gene produced by the processes of Figs. 1 and 4-7.

Fig. 3 shows the intercistronic region between two IFN-B' genes tandemly arranged in accordance with the processes of Figs. 1 and 4-5. Eleven base pair 5' to the second IFN-B' gene is a ribosome-binding site marked by the heavy bar. The initiation codon for the second IFN-B' gene is marked by a light bar.

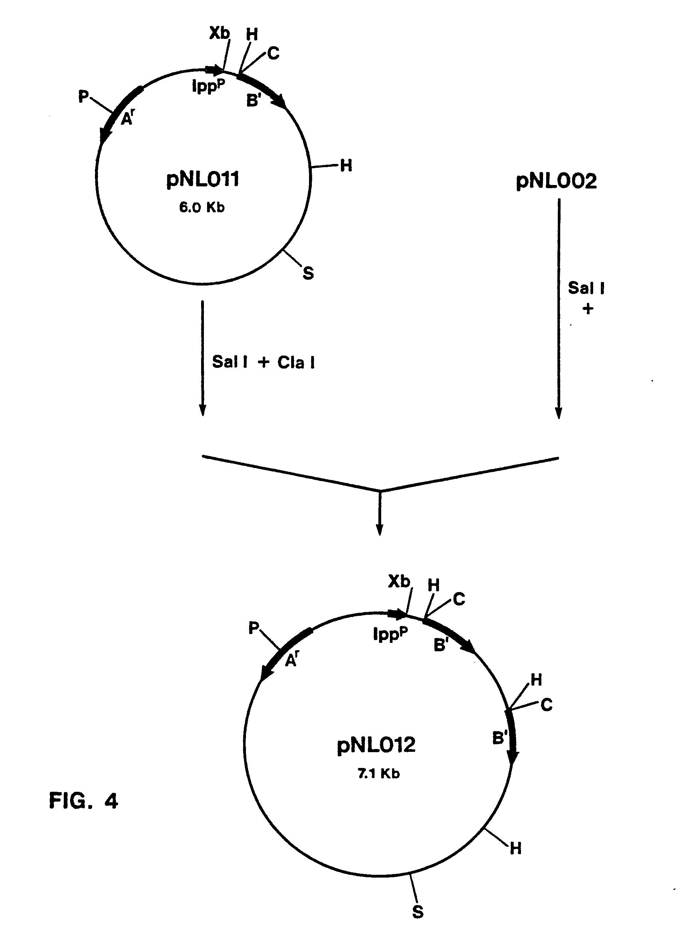

Fig. 4 shows the construction of hybrid plasmid pNL012 which contains two linked IFN-B' genes. pNL011 is similar to pNL001 except that the lac promoter operator region is removed so that transcription is solely controlled by the lipoprotein promoter and transcription therefore becomes constitutive. Abbreviations used:

$lpp^p$ = lipoprotein promoter; B', Xb, H, C, S, P, and $A^r$, same as in Fig. 1.

Fig. 5 shows the construction of hybrid plasmids pNL026 and pNL027 which contain three linked IFN-B' genes and four linked IFN-B' genes, respectively. The abbreviations used are the same as in Fig. 4.

Fig. 6 shows the construction of hybrid plasmid pNL022. pNL022 is similar to pNL011 except that a XhoI site is located 5' to the lipoprotein promoter. Abbreviations used: Xh = XhoI; remainder as in Fig. 4.

Fig. 7 shows the construction of hybrid plasmids pNL024-1 and pNL024-2. These plasmids each contain two promoter-gene units. Abbreviations as in Fig. 6.

Figs. 8-10 schematically illustrate the automatic reading direction alignment aspects of the present invention.

## DESCRIPTION OF THE PREFERRED EMBODIMENTS

As described above, the present invention relates to genetically engineered hybrid nucleic acid sequences which, for the same number of genes, have a smaller overall size than genetically engineered prior art sequences. As used herein, the term "hybrid nucleic acid sequence" is intended to include all types of genetically engineered nucleic acid sequences which include structural genes coding for one or more selected polypeptide sequences and which can be used to transform a host organism so that the organism will produce the selected polypeptide sequences. The term is thus intended to include, without limitation, hybrid nucleic acid sequences in the form of hybrid plasmids, artificial chromosomes, transposable elements which can be introduced into the endogenous chromosomes of a host cell, cosmids, and the like.

The reduction in hybrid nucleic acid sequence size is achieved by placing a plurality of tandemly arranging genes under the control of a single promoter and a single transcriptional terminator, each recognized by the host cell. Prior art hybrid nucleic acid sequences, in particular, hybrid plasmids, included a promoter and a transcriptional terminator for each structural gene carried by the plasmid. As demonstrated by the present invention, this redundant arrangement can be eliminated and substantial savings in overall sequence size thus achieved.

As an illustration of the magnitude of the size reduction which can be achieved by the present invention consider the situation wherein the promoter region has a length of 0.23 Kb, the structural gene of interest and its associated translation initiation and termination sites can be obtained by restriction enzyme techniques as a unit having a length of 1.1 Kb, and the transcription termination region has a length of 1.0 Kb. These values correspond to the length of the promoter, the structural gene for human leukocyte interferon, and the transcription termination region used in the examples below.

A hybrid nucleic acid sequence constructed in accordance with the prior art techniques and containing "n" copies of the structural gene would have a length of n x 2.33 Kb. In contrast, a hybrid nucleic acid sequence having the same number of genes but constructed in accordance with the present invention would have a length of 1.23 + n x 1.1 Kb. This represents a savings of (n - 1) x 1.23 Kb, i.e., 1.23 Kb for n = 2; 2.46 Kb for n = 3; 3.69 Kb for n = 4; etc. In the limit, as "n" becomes very large, the prior art hybrid nucleic acid sequence will be over twice as long as the hybrid nucleic acid sequence of the present invention for the same number of genes ([nx2.33] / [1.23+nx1.1] → 2.1).

If the length of the unit carrying the structural gene can be made smaller, e.g., by using different sets of restriction enzymes, or other enzymes or techniques known to the art to construct the unit, the savings become even greater. For example, by reducing the length of the structural gene unit from 1.1 Kb to 0.56 Kb, the ratio for large "n" of the length of the prior art nucleic acid sequence to the length of the nucleic acid sequence of this invention increases from 2.1 to 3.2. This means that for the same number of genes, the hybrid nucleic acid sequences of the present invention will have a length which is less than a third of the length the sequence would have if constructed in accordance with the prior art techniques.

In addition to preparing hybrid nucleic acid sequences having multiple copies of the same gene, the present invention can also be used to prepare sequences having a group of structural genes under the control of a single promoter and a single transcription terminator. Such sequences can be used to simultaneously produce selected polypeptides in selected ratios based on the relative number of genes coding for each polypeptide included in the sequence. For example, a hybrid sequence can be constructed having structural genes for two or more different interferons, such as, two or more alpha interferons which in combination may produce a synergistic therapeutic effect. Through adjustments in the copy number within the sequence of each of the two genes, the relative amounts of the two interferons produced can be varied as desired. Other examples of desirable co-production applications include the simultaneous production of the components of biologically active molecules, such as, the production of insulin A and insulin B, the production of the components of an immunoglobulin, and the like.

The preparation of the hybrid nucleic acid sequences of the present invention involves the steps of preparing or selecting sub-sequences which, when combined, will produced an overall sequence including inter alia: 1) a promoter region; 2) two or more structural gene coding regions, each of which includes a ribosome-binding site, a translation initiation site and a translation termination site; and 3) a transcription termination region.

Most conveniently, the overall sequence is constructed by preparing or selecting a set of ligatable fragments and then ligating those fragments with a ligation enzyme to produce the desired overall sequence. The set of fragments, of course, must be chosen so that their combination will, in fact, produce the desired overall sequence. The fragments can be obtained in various ways known to persons skilled in the art, including by means of restriction enzymes and various other enzymes used in the art for gene manipulation, and by means of site specific mutagenesis. Once obtained, the fragments can be ligated together in a variety of ways, such as, through the use of various ligases known to the art.

Conceptually, the simplest set of fragments which can be used to construct the hybrid nucleic acid sequences of the present invention is as follows: a first fragment having a promoter region at one end and a transcription terminator at the other end, the reading direction of the fragment being from the end having the transcription terminator towards the promoter, and a group of second fragments, each fragment having a ribosome-binding site, a translation initiation region, a structural gene coding region and a translation termination region, the reading direction of each fragment being from the ribosome-binding site towards the translation termination region. As will be evident to persons skilled in the art, either the first fragment or an additional separate fragment will normally include a replication origin and a selective marker so that the the hybrid nucleic acid sequence will be replicated and so that host cells which have been transformed with the hybrid sequence can be distinguished from host cells which have not been transformed.

Assuming each fragment has appropriate ligatable ends, the ligation of the first fragment with the group of second fragments will produce final sequences which have the desired "promoter -- multiple structural genes -- transcription terminator" form. Although the form will be correct, the reading directions of the various fragments may not all be aligned. Solution of this alignment problem is critical to the success of the present invention, but fortunately, as discussed below, the problem can be solved in a number of ways.

Although construction of the final sequence from the above set of fragments is conceptually easiest to see and in many cases can be used to prepare the hybrid nucleic acid sequences of this invention, in other cases it may be more convenient, e.g., because of the types of restriction enzymes available and the source of the structural genes to be incorporated in the hybrid sequence, to use fragments having different contents and arrangements of contents. For example, in the specific examples described below, fragments of the following types have been used: a first fragment having, inter alia, a transcription terminator, a promoter, a ribosome-binding site, and a translation initiation site, the reading direction of the fragment being from the transcription terminator towards the translation initiation site, and a group of second fragments having, inter alia, a translation initiation site, a coding region for the desired structural gene, a translation termination site and a ribosome-binding site, the reading direction of these fragments being from the translation initiation site towards the ribosome-binding site.

Ligation of these fragments and alignment of their reading directions will again produce the desired "promoter -- multiple structural genes -- transcription terminator" arrangement, but in this case the first fragment carries the ribosome-binding site for the first structural gene, rather than the fragment which carries that gene carrying its own ribosome-binding site, and each structural gene fragment carries the ribosome-binding site for the next structural gene fragment rather than for itself. Note that with this arrangement there is an extra translation initiation site between the promoter and the coding region for the fir . structural gene. Since the translation process will respond to the first translation initiation site appearing after a ribosome-binding site, the polypeptide produced by the first structural gene will include some extra initial amino acids corresponding to the region after the first translation initiation site and before the coding region of the structural gene. Also, because the fragment for the last structural gene includes a ribosome binding site downstream of its translation termination site, it is possible that polypeptide sequences other than those defined by the selected structural genes will be produced by transformed hosts.

As will be evident to persons of ordinary skill in the art, the desired "promoter -- multiple structural genes -- transcription terminator" arrangement can be achieved by ligating fragments having a variety of configurations other than those described herein above.

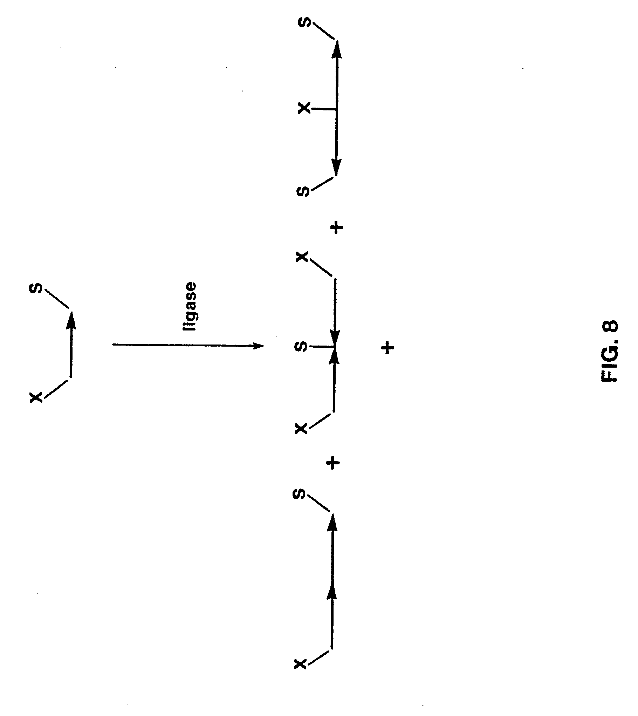

As discussed above, one of the critical steps in preparing hybrid nucleic acid sequences having multiple genes under the control of a single promoter and a single transcription terminator involves arranging the various fragments so that their reading directions all have the same orientation. The problem is illustrated schematically in Figure 8.

For random recombination, as the number of fragments being combined increases, the number of combinations having mis-oriented fragments increases exponentially. For example, for two genes, the chances of randomly obtaining a recombinant wherein both genes have the same reading direction is one out of two. For three genes, it drops to one out of four; and for four, it becomes one out of eight. Selection of those recombinants having aligned reading directions can be determined on a functional basis, i.e., by transforming host cells and looking for those cells which produce the desired product in large amounts corresponding to a high copy number for the gene. Plainly, this is a tedious and time consuming process.

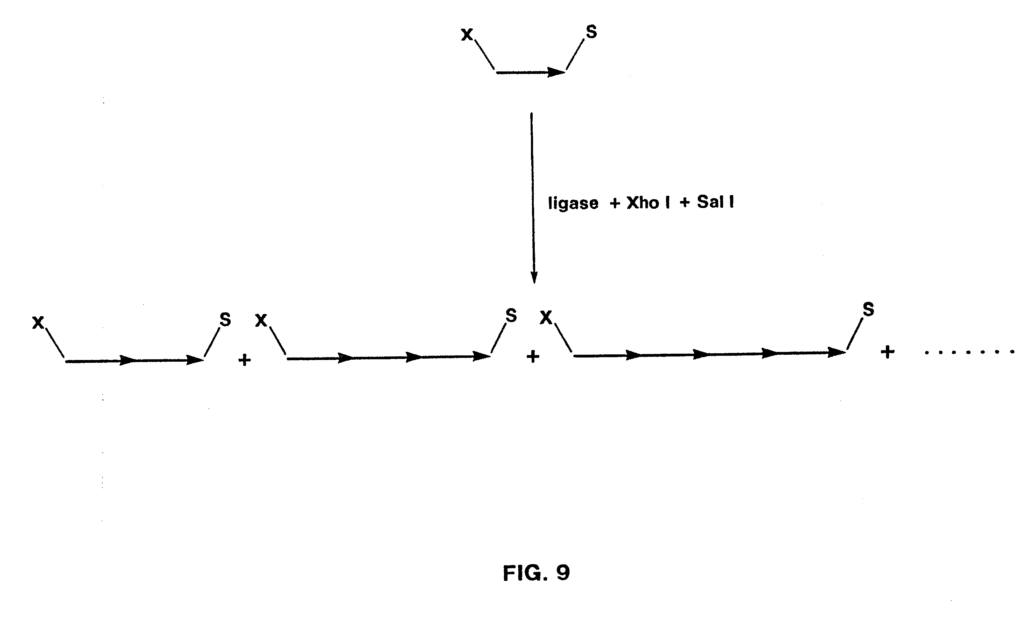

Rather than using this brute force approach, it has now been found that the reading directions of the fragments making up the hybrid nucleic acid sequence can be aligned essentially automatically as the sequence is formed if the ligatable ends of the fragments are properly selected. Specifically, in accordance with one embodiment of this aspect of the invention (see Figure 9), each fragment is given a first type of ligatable end at its upstream end and a second type of ligatable end at its downstream end. The first and second types of ligatable ends are chosen so that the combination of a first type with itself can be cleaved by a first restriction enzyme, and the combination of a second type with itself can be cleaved by a second restriction enzyme. The ligatable ends are further selected so that the combination of a first and second type is substantially immune from cleavage by either the first or second type of restriction enzyme.

With this choice of ligatable ends, all that is required to align reading directions is treatment of the ligated fragments with the first and second restriction enzymes. Such treatment will automatically cleave the misalignments (upstream/upstream = first type/first type and downstream/downstream = second type/second type) while leaving essentially unaffected the proper alignments (downstream/upstream = second type/first type). The alignment producing treatment can be done simultaneously with ligation of the fragments by subjecting the fragments to both ligation enzymes and the first and second restriction enzymes, or, alternatively, the ligation and alignment steps can be performed separately, the fragments first being ligated, then aligned, then ligated again, and so forth.

Various combinations of first and second type ligatable ends can be used with the present invention. Particularly preferred choices include the combination of a ligatable end produced by a XhoI restriction enzyme and a ligatable end produced by a SalI restriction enzyme, and the combination of a ligatable end produced

by a BclI restriction enzyme and a ligatable end produced by a BamHI restriction enzyme. In the former case, the first and second restriction enzymes used to produce the alignment are XhoI and SalI, while in the latter case, they are BclI and BamHI. Either member of the combination can serve as the upstream or downstream ligatable end provided that all fragments have the same upstream and downstream ends. Other combinations of ligatable ends which can be used in practicing this aspect of the present invention, both presently known and as may be developed in the future, will be evident to persons skilled in the art.

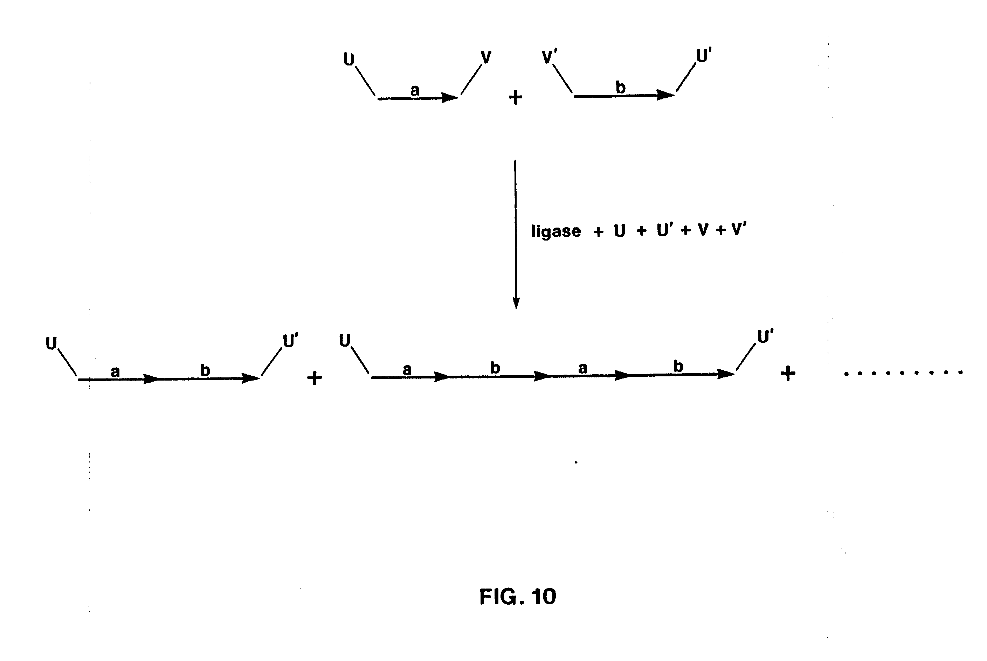

An additional embodiment of the self-alignment aspect of the present invention is illustrated in Figure 10. In this case, two different types of sequences are to be combined, e.g., a first type carrying one structural gene and a second type carrying a second structural gene. For this situation, the ligation procedure should not only control the orientation of the reading directions of the segments, but, preferably, should also control the amount of each type of segment which becomes combined. The procedure shown in Figure 10 achieves both of these goals.

Specifically, in accordance with this embodiment of the invention, the sequences of the first type ("a" in Figure 10) are constructed to have a ligatable end U of a first type, e.g., XhoI, at their upstream ends and a ligatable end V of a second type, e.g., BclI, at their

downstream ends. The ligatable ends of the first and second types can ligate with themselves (UU or VV), but not with each other (UV). In each case, self-ligations are cleavable by appropriate restriction enzymes U and V, e.g., XhoI and BclI.

Similarly, the sequences of the second type (b) are constructed to have a ligatable end V' of a third type, e.g., BamHI, at their upstream end and a ligatable end U' of a fourth type, e.g., SalI, at their downstream end. Again, these ligatable ends can ligate with themselves, but not with each other. Also, self-ligations are cleavable by appropriate restriction enzymes U' and V', e.g., BamHI and SalI.

To achieve the twin goals of reading direction alignment and control of the relative amounts of the two types of sequences combined, the ligatable ends of the first, second, third, and fourth types are related as follows: the ligatable ends of the first type (U) can ligate with the ligatable ends of the fourth type (U'), but not with the ligatable ends of the third type (V'), and the ligation with the ligatable ends of the fourth type (UU') is essentially immune from cleavage by any of the first, second, third, or fourth restriction enzymes; the ligatable ends of the second type (V) can ligate with the ligatable ends of the third type (V'), but not with the ligatable ends of the fourth type (U'), and the ligation with the ligatable ends of the third type (VV') is essentially immune from cleavage by

0154576

any of the first, second, third or fourth restriction enzymes.

As shown in Figure 10, for ligatable ends having these characteristics, ligation with a ligase, in combination with treatment with the first, second, third and fourth restriction enzymes, automatically results in reading direction alignment and a hybrid sequence consisting of alternating first and second sequences (ababab...), i.e., controlled relative amounts of each sequence.

If each of the two sequences carries one copy of a structural gene, the hybrid sequence will carry essentially equal numbers of each of the genes. On the other hand, if one of the sequences carries, for example, two copies of its structural gene, while the other sequence only carries one copy, the hybrid sequence will essentially carry twice the number of first genes as second genes. A similar gene ratio can be achieved by providing one of the sequences in two forms: a first form with first and second type ligatable ends, and a second form with third and fourth type ligatable ends. In this case, the hybrid sequence will include adjacent repeats of the sequence provided in two forms and thus will, on average, contain more of the gene or genes carried by that sequence. As will be evident to persons skilled in the art, using strategies such as these, essentially any gene ratio desired can be achieved.

0154576

Once ligation and alignment of reading directions has been completed, the hybrid nucleic acid sequence is ready for use in transforming an appropriate microorganism so as to produce the product or products defined by the structural genes. Techniques for such transformation, subsequent culturing of the transformed organism and collection of the desired products are well known in the art. See, for example, Molecular Cloning: A Laboratory Manual, by T. Maniatis et al, Cold Spring Harbor Laboratory, Cold Spring Harbor, N.Y. (1982). Suitable hosts for the hybrid sequences include both prokaryotic and eukaryotic cells. In general, the host cell must be one which will recognize the functional elements making up the hybrid nucleic acid sequence, that is, the promoter, the transcription terminator, the ribosome-binding sites, the translation initiation sites, and the translation termination sites.

Without intending to limit it in any manner, the present invention will be more fully described by the following examples wherein hybrid plasmids containing up to four copies of a human interferon gene under the control of a single promoter and a single transcription terminator are constructed. For comparison, hybrid plasmids are also constructed which contain multiple copies of the same gene but with each gene having its own promoter and its own transcription terminator. To facilitate discussion of the various plasmids, those plasmids having multiple copies of the same gene under the control of one promoter and one transcription terminator will be referred to as "homopolycistronic", while those having multiple promoter-gene-terminator sequences will be referred to as "polymonocistronic", where "cistron" has its usual meaning of a structural gene and its associated translation initiation and termination sites.

## MATERIALS AND METHODS

### Strains and Plasmids

E. coli K-12 strains JA221 (hsd M+ hsd R- recA Len Lac Y trp) and JA221 (hsd M+ hsd R- recA Len Lac Y trp/F'lac I$^q$ lac Z+ lac Y+ pro A+ pro B+) were used as transformation hosts.

The cloning vehicles pIN-II-A3, pIN-I-A3 (pKEN037(A3)), pKEN030 and pCGS261 were used in preparing the novel hybrid nucleic acid sequences of the present invention. Construction of the first vehicle is described in Nakamura, K. and Inouye, M. (1982) EMBO J., Vol.1, pages 771-775. Construction of the second and third vehicles is described in U.S. patent applications serial numbers 222,010 and 286,070, filed January 2, 1981 and July 23, 1981, respectively, and published on July 28,1982 as UK patent application serial number GB 2,091,269 A. Construction of the fourth vehicle is described in U.S. patent application serial number 418,521, filed September 15, 1982. The pertinent portions of these patent applications and literature reference are incorporated herein by reference.

With regard to vehicle pCGS261, this plasmid contains a leukocyte interferon gene (LeIFN-B', or, as it is also known, IFNα8') for expression in yeast under the control of a yeast galactose promoter. In this construction, a 69 bases DNA sequence thought to code for the signal sequence of the interferon protein has been removed and replaced by an ATG translation initiation codon. The nucleotide sequence of the coding region of the LeIFN-B reported by Goeddel et. al. (see Goeddel, D., Leung, D., Dull, T., Gross, M., Lawn, R., McCandliss, R., Seeburg, P., Ullrich, A., Yelverton, E., and Gray, P. (1981) Nature, 290:20-26.) is identical to this leukocyte interferon gene, except for a single base deletion and, twelve bases away, a single base insertion which cause the sequence coding for the 99th to 102nd amino acid residues to be changed from Val-Leu-Cys-Asp to Ser-Cys- Val-Met. Cultures containing plasmid pCGS261 are on deposit with the American Type Culture Collection, 12301 Parklawn Drive, Rockville, Maryland. These cultures are identified by Accession Number 20644, Strain Designation CGY144 and were deposited by Collaborative Research, Inc., Waltham, Massachusetts.

Plasmids were isolated by the method described by Tanaka & Weisblum. Tanaka, T. and Weisblum, B. (1975) J. Bacteriol., 121:354-362.

Enzymes

All restriction enzymes, $T_4$ DNA ligase and DNA polymerase I Klenow fragment were obtained from New England Bio Labs. Bacterial alkaline phosphatase was obtained from Worthington Diagnostic Systems, Inc., Freehold, N.J. Nuclease $S_1$ was obtained from Boehringer-Mannheim, Indianapolis, Ind.

## Preparation of Total Lysate

One ml of E. coli JA221 cells containing the plasmids to be assayed were grown in L-Broth containing 50 ug/ml ampicillin. For inducible systems (see below), at an optical density of 0.4 at 600 nm, isopropyl-β-D-thiogalactoside (IPTG, Sigma) was added to a final concentration of 2mM. After incubation of the culture at 37°C for 1 hour, cells were sedimented and resuspended on ice in 5M guanidine chloride containing 0.5mM phenyl-methanesulfonyl fluoride (PMSF, Sigma) for 10 minutes with vigorous vortexing. The % of breakage was checked using a light microscope. The extracts were obtained after centrifugation. For constitutive systems, cells were harvested at optical density of 0.5 to 1.0 at 600 nm.

## Measurement of Antiviral Activity

Antiviral assays were performed using cytopathic effect inhibition (CPE) of Vesicular Stomatitis Virus on Hep-2 cells. All interferon titers were determined relative to the NIH human leukocyte interferon reference standard.

## Gel Electrophoresis

Agarose gel electrophoresis was performed as described by Sharp, et. al. See Sharp, P.A., Snyder, B. and Sambrode, J. (1973) Biochemistry 12:3055-3063. Fragments were isolated from the gel by electroelution onto a Whatman NA-45 paper strip and the fragment was recovered from NET buffer (1M NaCl, 0.1mM EDTA, 20mM Tris-

28

0154576

HCl, pH 8). The recovery was found to be greater than 90% and contamination of the final product by agarose was not found.

Example 1

Preparation of Homopolycistronic Hybrid Plasmids

Following the procedure shown in Fig. 1, a 1.1 Kb HindIII fragment containing the IFN-B' gene from pCGS261 was cloned into the HindIII site of the pIN-II-A3 cloning vehicle for subsequent expression in E. coli under the control of an E. coli outer membrane lipoprotein promoter and a lactose promoter-operator. The resulting hybrid plasmid contained one copy of the LeIFN-B' gene and was designated pNL001.

As also shown in Fig. 1, a 2.2 Kb DNA fragment, which had two 1.1 Kb HindIII fragments linked in the same orientation, was cloned into the HindIII site of pIN-II-A3. This hybrid plasmid was designated pNL002. By checking restriction sites, the orientation of the LeIFN-B' genes in pNL001 and pNL002 was determined to be the same as that of the lipoprotein promoter and lactose promoter-operator.

Fig. 2 shows the N-terminus of the coding region immediately after the lactose promoter-operator for pNL001 and pNL002. As can be seen in that figure, the interferon coding region is preceded by 10 amino acid residues which are introduced by pIN-II-A3 and the HindIII and ClaI restriction sites. The ribosome-binding site is 7 bases 5' to the first ATG. For pNL002, as shown in Fig. 3, the translation termination site (TGA) of the first LeIFN-B' is separated from the translation initiation site (ATG) of the second LeIFN-

0154576

B' by 636 bases. There is a ribosome-binding site 12 bases 5' to the ATG of the second LeIFN-B'.

Due to the lac promoter-operator element they contain, pNL001 and pNL002 are both inducible systems. In order to obtain constitutive systems which do not need inducer for expression, pNL011 was constructed from pIN-I-A3 and pCGS261. pIN-I-A3 is exactly the same as pIN-II-A3 except it lacks the lac-promoter- operator. Following the procedure shown in Fig. 1, pNL011 was formed from pIN-I-A3 and pCGS261 in the same manner as pNL001 was formed from pIN-II-A3 and pCGS261. As with pNL001, pNL011 contains only a single copy of LeIFN-B'.

To obtain a constitutive system having two copies of LeIFN-B', a 3.1 Kb fragment was obtained from SalI digestion and ClaI partial digestion of pNL002 and ligated to a 4.0 Kb fragment from SalI and ClaI digestion of pNL011. The resulting plasmid was designated pNL012. See Fig. 4. By restriction site mapping, the two LeIFN-B' genes were found to be in the same orientation as the lpp promoter.

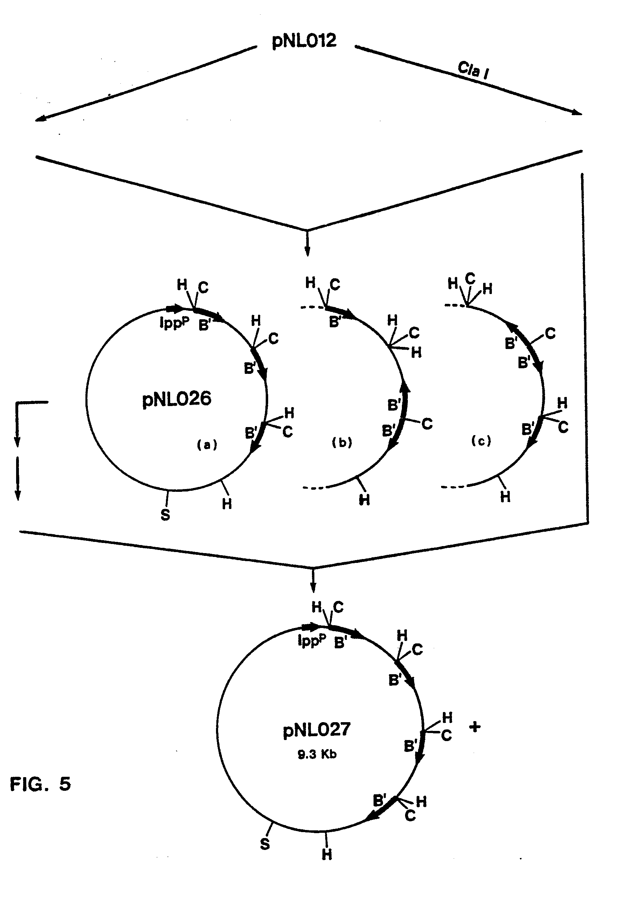

Using pNL012 as starting material, plasmids containing tri-cistronic and tetracistronic LeIFN-B' genes were constructed as shown in Fig. 5. After partial digestion with ClaI, 7.1 Kb DNA fragments which were pNL012 linearized by a single cut at either one of the two possible ClaI sites were isolated and ligated with a 1.1 Kb LeIFN-B'-containing ClaI fragment obtained from pNL012. The resulting plasmids can have three possible structures. After transformation in _E. coli_ JA221, the plasmids from each clone were purified and checked with restriction enzymes. The plasmid with all three LeIFN-B' genes in the same direction was designated pNL026. pNL027, with four LeIFN-B' genes in the same direction was similarly obtained.

0154576

## Example 2

## Preparation of Polymonocistronic Hybrid Plasmids

For the construction of a polymonocistronic hybrid plasmid, it was necessary to have convenient restriction sites 5' to the promoter and 3' to the transcription termination site. The nature of the restriction sites were chosen so that they would facilitate the formation of tandem repeats, control the relative orientation of the tandem repeats, and be suitable for checking the size of the tandem formed.

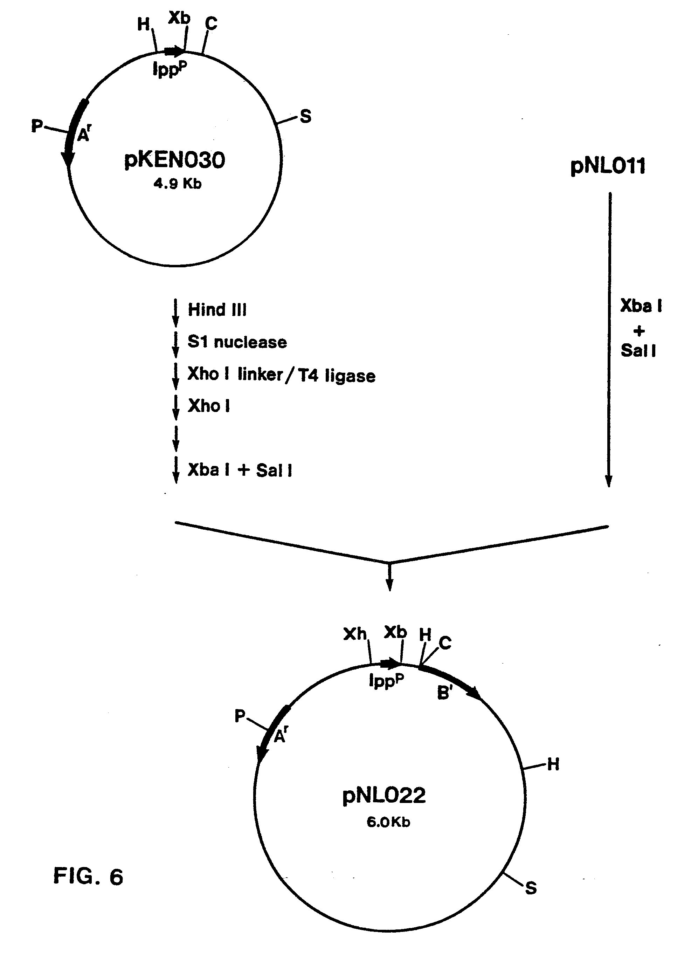

For this purpose, pNL022 was constructed in accordance with the procedures shown in Fig. 6. This plasmid has a XhoI site 5' to the lipoprotein promoter and a SalI site 3' to the transcriptional terminator. pKEN030 which has a HindIII site 5' to the lipoprotein promoter was used as the starting material. pKEN030 was linearized by a HindIII restriction enzyme, and made blunt ended by S1 nuclease. XhoI linkers were then attached by blunt-end ligation, cohesive ends formed by XhoI cleavage, and the DNA was recircularized by ligation. After transformation and screening, the resultant plasmid was cut by XbaI and SalI, and the large fragment obtained was ligated to the small XbaI-SalI fragment from pNL011 to form pNL022. Other than the additional XhoI site, pNL022 is exactly the same as pNL011.

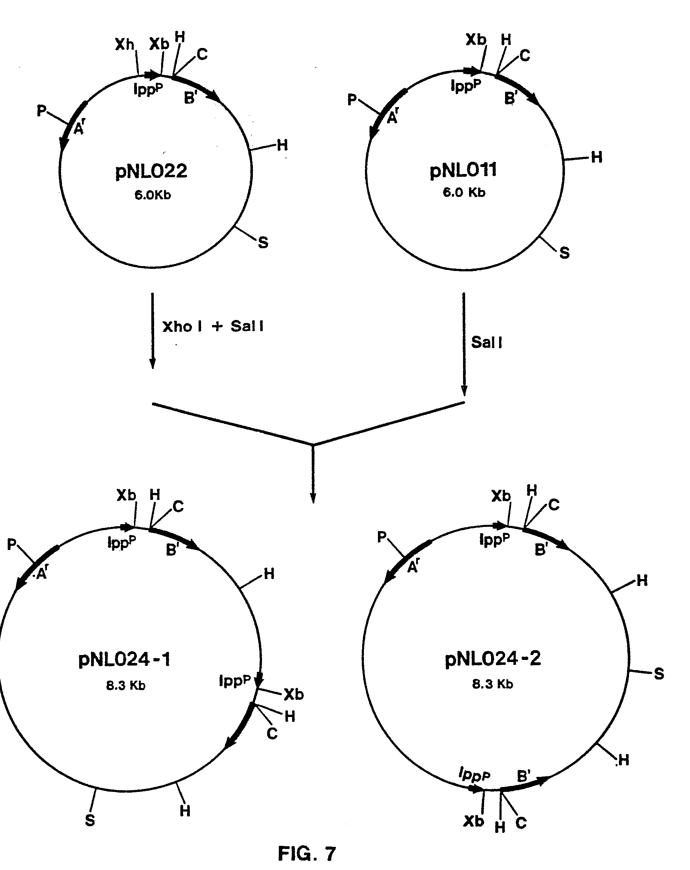

To form the desired polymonocistronic hybrid plasmid, pNL022 was cut by XhoI and SalI and the resulting small fragment was ligated to pNL011 linearized

0154576

by SalI. See Fig. 7. The two possible hybrid plasmids with opposite orientation were designated pNL024-1 and pNL024-2. Since the joining of a SalI cohesive end with another SalI cohesive end will re-create a SalI site, while joining of a SalI cohesive end and a XhoI cohesive end will result in no restriction site, pNL024-1 was easily distinguished from pNL024-2 by checking sizes of restriction fragments, the SalI sites of the two plasmids being located at different positions.

So that polymonocistronic hybrid plasmid pNL024-1 could be compared with an equivalent homopolycistronic hybrid plasmid, an additional homopolycistronic hybrid plasmid, designated pNL023, was constructed in the same way as pNL022 (see Fig.6), except that pNL012 instead of pNL011 was used as starting material.

0154576

## Example 3

## Automatic Reading Direction Alignment

## Of Nucleic Acid Sequences

To demonstrate the automatic reading direction alignment aspects of the present invention, pNL022, prepared in accordance with Figure 6, was cleaved by XhoI and SalI and a 2.3 Kb fragment was isolated and self-ligated by T4 ligase and treated with XhoI and SalI. The ligation mixture was subjected to gel electrophoresis. Electrophoresis bands corresponding to DNA molecules larger than 6 Kb were eluted and treated with SalI and XhoI restriction enzymes, and the mixture was again subjected to gel electrophoresis. Electrophoresis results showed that SalI and XhoI treatment did not alter the gel pattern. This result indicates that these DNA molecules essentially consist of 2.3 Kb fragments tandemly aligned in the same orientation.

0154576

<div align="center">

Example 4

Interferon Titers Produced by

Homopolycistronic and Polymonocistronic

Hybrid Plasmids

</div>

The interferon activities of E. coli cells (JA221 strain) harboring various plasmids were determined by cytopathic effect inhibition (CPE) of Vesicular Stomatitis Virus on Hep-2 cells.

For the homopolycistronic series pNL011 (mono-cistron), pNL012 (di-cistron), pNL026 (tri-cistron), and pNL027 (tetra-cistron), the interferon activities were $2.10 \times 10^4 \pm 7.82 \times 10^3$, $5.03 \times 10^4 \pm 1.64 \times 10^4$, $6.85 \times 10^4 \pm 2.69 \times 10^4$, and $9.46 \times 10^5 \pm 3.17 \times 10^4$ units/ml of cell culture/O.D., respectively, that is, in the relative ratio of 1.0:2.4:3.3:4.5. For the pNL022 (mono-cistron) and pNL023 (di-cistron) series, the interferon activities were $8.04 \times 10^4 \pm 4.6 \times 10^4$ and $1.60 \times 10^5 \pm 6.7 \times 10^4$ units/ml of cell culture/O.D., respectively, that is, in the relative ratio of 1:1.99. This ratio is similar to that of pNL011 and pNL012, however, the titers of the pNL022 and pNL023 series were found to be consistently higher than the titers of the pNL011 and pNL012 series by a factor of about three. It is believed that this difference is due to the presence of XhoI sites in the pNL022 and pNL023 plasmids.

Plasmid pNL024-1, which is a polymonocistronic plasmid (di-cistronic), was found to have a level of

0154576

expression (1.47 x $10^5$ ± 6.23 x $10^4$ units/ml of cell culture/O.D.) about the same as the comparable homopolycistronic plasmid pNL023 (1.60 x $10^5$ ± 6.7 x $10^4$ units/ml of cell culture/O.D.).

The interferon titers found for cells harboring the various homopolycistronic and polymonocistronic plasmids, especially those containing more than one interferon B' gene, were found to compare very favorably with those reported by Yelverton, et. al., for LeIFN-B, although it should be noted that for interferons made by the first structural gene downstream from the promoter there were nine extra amino acids at the amino terminus. See Yelverton, E., Leung, D., Weck, P., Gray, P., and Goeddel, D. (1981) Nucleic Acids Research, 9:731-741. Elimination of these extra amino acids may produce even higher titers.

In combination, the foregoing results clearly demonstrated that 1) the gene dosage effect occurs in homopolycistronic systems, and 2) the level of expression is about the same for comparable homopolycistronic and polymonocistronic systems, that is, systems having the same number of copies of a structural gene per plasmid. As discussed in detail above, these two phenomena allow for the optimization of two contradictory factors, that is, the maximization of the copy number of a cloned gene in a plasmid coupled with the minimization of the plasmid size in order to maintain high plasmid copy number and transformation efficiency. In short, by adopting the homopolycistronic format, plasmid size can be reduced without sacrificing gene dosage. With this flexibility, the optimal copy number of genes to be incorporated into a plasmid to obtain maximum product production can easily be established experimentally.

Even greater packing density than that described in the foregoing examples can be achieved by shortening the intercistronic region. In naturally occurring multicistronic operons, intercistronic regions typically range from a few to several hundred base pairs. See Christie, G.E. and Platt, T. (1980) *J. Mol. Biol.*, *142*:519-530, and Selker, E. and Yanofsky, C. (1979) *J. Mol. Biol.*, *130*:135-143. As shown in Fig. 3, for the plasmids of the foregoing examples, there is a 636 base pair intercistronic region. Accordingly, more than 600 base pairs of intercistronic region may be deleted by methods such as Bal 31 digestion or site specific mutagenesis.

Although specific embodiments of the invention have been described and illustrated, it is to be understood that modifications can be made without departing from the invention's spirit and scope. For example, other types of hybrid nucleic acid sequences designed to produce other types of products than those illustrated can be constructed in accordance with the invention. Moreover, numerous optimizations of the hybrid sequences can be made within the scope of the invention. For example, the intercistronic regions, including the ribosome-binding sites, can be optimized to maximize translation reinitiation efficiency while still keeping the overall plasmid size small. Similarly, portions of the plasmid other than those carrying the tandemly arranged genes can be optimized to enhance product production. For example, if a par gene is incorporated into a hybrid plasmid, the size of the plasmid can be increased without sacrificing stability, and thus the total number of desired genes which the plasmid can carry can be increased. Optimizations of these and similar types are within the scope of the present invention.

Microoganisms and recombinant nucleic acid sequences prepared in accordance with the present invention are exemplified by a culture deposited in the culture collection of the American Type Culture Collection, Rockville, Maryland on January 27, 1984, and assigned ATCC accession number 39589. This culture corresponds to plasmid pNL023 transposed into E. coli. strain JA221 and is designated ISE023.

What is claimed is:

1. A hybrid nucleic acid sequence for use in producing one or more selected polypeptides in a host cell comprising: a promoter sequence recognized by the host cell at one end; a transcription terminator sequence recognized by the host cell at the other end; and between the promoter sequence and the transcription terminator sequence: a first region which includes a ribosome-binding site, a translation initiation site, a coding region for one of said one or more selected polypeptides, and a translation termination site, but which does not include the promoter sequence or the transcription terminator sequence; and one or more second regions, each of which includes a ribosome-binding site, a translation initiation site, a coding region for the same or a different one of said one or more selected polypeptides, and a translation termination site, and which does not include the promoter sequence or the transcription terminator sequence, the reading directions of the promoter sequence, the transcription terminator sequence, the first region and each of the second regions having the same orientation.

2. The hybrid nucleic acid sequence of Claim 1 wherein at least one of said one or more selected polypeptides displays an immunological or biological activity of human leukocyte interferon.

3. A transformed host cell which includes the hybrid nucleic acid sequence of Claim 1.

4. A polypeptide or fragments and derivatives thereof produced by the transformed host cell of Claim 3.

5. A method for synthesizing one or more selected polypeptides in a microorganism comprising the steps of:

(a) transforming the microorganism with a hybrid nucleic acid sequence comprising: a promoter sequence recognized by the microorganism at one end; a transcription terminator sequence recognized by the host cell at the other end; and between the promoter sequence and the transcription terminator sequence: a first region which includes a ribosome-binding site, a translation initiation site, a coding region for one of said one or more selected polypeptides, and a translation termination site, but which does not include the promoter sequence or the transcription terminator sequence; and one or more second regions, each of which includes a ribosome-binding site, a translation initiation site, a coding region for the same or a different one of said one or more selected polypeptides, and a translation termination site, and which does not include the promoter sequence or the transcription terminator sequence, the reading directions of the promoter sequence, the transcription terminator sequence, the first region and each of the second regions having the same orientation;

(b) culturing the transformed microorganism; and

(c) collecting the one or more selected polypeptides.

6. The method of Claim 5 wherein at least one of said one or more selected polypeptides displays an immunological or biological activity of human leukocyte interferon.

7. A polypeptide or fragments and derivatives thereof produced by the method of Claim 5.

8. A method for producing a hybrid nucleic acid sequence wherein the coding regions for one or more selected polypeptides are tandemly arranged and placed under the control of a common promoter sequence and a common transcription terminator sequence, comprising the steps of:

(a) preparing or selecting first nucleic acid sequences which do not include the promoter sequence or the transcription terminator sequence, which have ligatable ends, and which include: (1) a region which codes for one of the one or more selected polypeptides; (2) a translation initiation site; (3) a translational termination site; and (4) a ribosome-binding site;

(b) ligating the first nucleic acid sequences to form second nucleic acid sequences which have ligatable ends and which include tandemly arranged coding regions for the one or more selected polypeptides;

(c) preparing or selecting third nucleic acid sequences which have ligatable ends and which include the promoter sequence and the transcription terminator sequence;

(d) ligating the second nuclei acid sequences with the third nucleic acid sequences to form fourth nucleic acid sequences; and

(e) selecting those fourth nucleic acid sequences in which each of the regions coding for one of said one or more selected polypeptides, each of the translation initiation sites, each of the translation termination sites, each of the ribosome-binding sites, the promoter sequence, and the transcription terminator sequence have the same reading direction.

9. The method of Claim 8 wherein the step of selecting the fourth nucleic acid sequences includes the steps of:

preparing or selecting first nucleic acid sequences which at one end have a ligatable end of a first type and at the other end have a ligatable end of a second type, the reading direction of the first nucleic acid sequences being from the ligatable end of the first type towards the ligatable end of the second type, the union of two ligatable ends of the first type being susceptible to cleavage by a first restriction enzyme, the union of two ligatable ends of the second type being susceptible to cleavage by a second restriction enzyme, the union of a ligatable end of the first type with a ligatable end of the second type not being substantially susceptible to cleavage by either the first or the second restriction enzyme; and

treating the second nucleic acid sequences with the first and second restriction enzymes.

10. The method of Claim 9 wherein the step of selecting the fourth nucleic acid sequences includes the further steps of:

preparing or selecting third nucleic acid sequences which at one end have a ligatable end of the first type and at the other end have a ligatable end of the second type, the reading direction of the third nucleic acid sequences being from the ligatable end of the first type towards the ligatable end of the second type; and

treating the fourth nucleic acid sequences with the first and second restriction enzymes.

11. The method of Claim 8 wherein at least one of said one or more selected polypeptides displays an immunological or biological activity of human leukocyte interferon.

12. A method for combining nucleic acid sequences so that their reading directions have the same orientation, comprising the steps of:

(a) preparing or selecting sequences which at one end have a ligatable end of a first type and at the other end have a ligatable end of a second type, the reading direction of a sequence being from the ligatable end of the first type towards the ligatable end of the second type, the union of two ligatable ends of the first type being susceptible to cleavage by a first restriction enzyme, the union of two ligatable ends of the second type being susceptible to cleavage by a second restriction enzyme, the union of a ligatable end of the first type with a ligatable end of the second type not being substantially susceptible to cleavage by either the first or the second restriction enzyme;

(b) ligating the sequences; and

(c) treating the ligated sequences with the first and second restriction enzymes.

13. A method for combining nucleic acid sequences of a first type with nucleic acid sequences of a second type to form hybrid sequences in which the reading directions of the the first and second sequences have the same orientation, and in which the number of first and second sequences are approximately equal, comprising the steps of:

(a) preparing or selecting sequences of the first type which at one end have a ligatable end of a first type and at the other end have a ligatable end of a second type, the reading direction of a sequence being from the ligatable end of the first type towards the ligatable end of the second type, the ligatable end of the first type not being substantially ligatable with the ligatable end of the second type, the union of two ligatable ends of the first type being susceptible to cleavage by a first restriction enzyme, the union of two ligatable ends of the second type being susceptible to cleavage by a second restriction enzyme;

(b) preparing or selecting sequences of the second type which at one end have a ligatable end of a third type and at the other end have a ligatable end of a fourth type, the reading direction of a sequence being from the ligatable end of the third type towards the ligatable end of the fourth type, the ligatable end of the third type not being substantially ligatable with the ligatable end of the fourth type, the ligatable end of the first type not being substantially ligatable with the ligatable end of the third type, the ligatable end of the second type not being substantially ligatable with the ligatable end of the fourth type, the union of two ligatable ends of the third type being susceptible to cleavage by a third restriction enzyme, the union of two ligatable ends of the fourth type being susceptible to cleavage by a fourth restriction enzyme, the union of a ligatable end of the first type with a ligatable end of the fourth type and the union of a ligatable end of the second type with a ligatable end of the third type not being substantially susceptible to cleavage by any of the first, second, third or fourth restriction enzymes;

(c) ligating the sequences of the first and second types with each other; and

(d) treating the ligated sequences with the first, second, third and fourth restriction enzymes.

FIG. 1

EcoR I      Hind III      Cla I      IFN-B'

----- ATG AAA GGG GGA ATT CCA AGC TTA TCG ATG TGT -----
      Met  Lys  Gly  Gly  Ile  Pro  Ser  Leu  Ser  Met  Cys

**FIG. 2**

IFN-B'      Hind III    Cla I    IFN-B'

----------- ... TGA ----------- ... TAGAAGGCCAAGCTTATCGATG -------------

**FIG. 3**

FIG. 4

FIG. 5

FIG. 6

FIG. 7

**FIG. 8**

ligase + Xho I + Sal I

**FIG. 9**

FIG. 10

**European Patent Office**

## EUROPEAN SEARCH REPORT

EP 85400191.4

## DOCUMENTS CONSIDERED TO BE RELEVANT

| Category | Citation of document with indication. where appropriate, of relevant passages | Relevant to claim | CLASSIFICATION OF THE APPLICATION (Int Cl 4) |
|---|---|---|---|
| A | EP - A1 - 0 060 057 (GENENTECH, INC.)  <br> * Claims 1-3,6,8 *  <br> ---- | 1,2 | C 12 N 15/00 <br> C 12 P 19/34 <br> C 12 P 21/00 <br> C 07 K 1/00 <br> A 61 K 45/02 |

### TECHNICAL FIELDS SEARCHED (Int. Cl.4)

C 12 N

C 12 P

C 07 K

A 61 K

The present search report has been drawn up for all claims

| Place of search | Date of completion of the search | Examiner |
|---|---|---|
| VIENNA | 13-05-1985 | WOLF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82